# DEMANDE DE BREVET EUROPEEN

(11) **EP 0 561 664 A1**
(43) Date de publication de la demande: **22.09.1993**
(21) Numéro de dépôt: 93400546.3
(22) Date de dépôt: 03.03.1993
(51) Int. Cl.: C07D 403/10, C07D 239/36

(54) **Procédé de préparation de 4-pyrimidinones**

(30) Priorité: 16.03.1992 FR 9203115
(71) Demandeur: SYNTHELABO, F-92350 Le Plessis Robinson (FR)
(72) Inventeur: Chekroun, Isaac, F-93800 Epinay (FR); Bedoya Zurita, Manuel, F-75003 Paris (FR); Ruiz-Montes, José, F-78200 Mantes La Jolie (FR); Rossey, Guy, F-78960 Voisins Le Bretonneux (FR)
(74) Mandataire: Thouret-Lemaitre, Elisabeth

(57) **Abrégé**

Nouveau procédé de préparation de 4-pyrimidinones de formule (I) 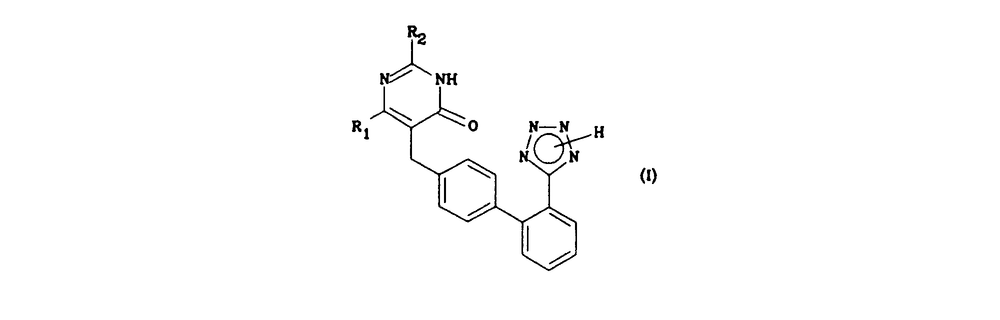
dans laquelle
R₁ représente soit un groupe (C₁₋₇)alkyle droit ou ramifié, soit un groupe (C₃₋₉)alcényle droit ou ramifié, soit un groupe cyclo(C₃₋₇)alkyl(C₁₋₆)alkyle,
R₂ représente soit un atome d'hydrogène, soit un groupe (C₁₋₇)alkyle droit ou ramifié, soit un groupe cyclo(C₃₋₇)alkyl(C₁₋₃)alkyle, soit un groupe aryl(C₁₋₃)alkyle éventuellement substitué sur le noyau, soit un groupe aryloxy(C₁₋₃)alkyle éventuellement substitué sur le noyau, soit un groupe arylthio(C₁₋₃)alkyle éventuellement substitué sur le noyau, soit un groupe arylsulfonyl(C₁₋₃)alkyle éventuellement substitué sur le noyau, soit un groupe hétéroaryl(C₁₋₃)alkyle éventuellement substitué sur le noyau.

## Description

La présente invention a pour objet un nouveau procédé de préparation de 4-pyrimidinones de formule (I) 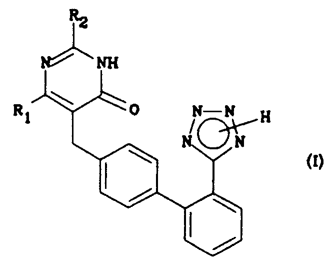
dans laquelle
R₁ représente soit un groupe (C₁ ₋ ₇)alkyle droit ou ramifié, soit un groupe (C₃ ₋ ₉)alcényle droit ou ramifié, soit un groupe cyclo(C₃ ₋ ₇)alkyl(C₁ ₋₆)alkyle,
R₂ représente soit un atome d'hydrogène, soit un groupe (C₁ ₋ ₇)alkyle droit ou ramifié, soit un groupe cyclo(C₃ ₋ ₇)alkyl(C₁ ₋ ₃)alkyle, soit un groupe aryl(C₁ ₋ ₃)alkyle éventuellement substitué sur le noyau, soit un groupe aryloxy(C₁ ₋ ₃)alkyle éventuellement substitué sur le noyau, soit un groupe arylthio(C₁ ₋ ₃)alkyle éventuellement substitué sur le noyau, soit un groupe arylsulfonyl(C₁₋₃)alkyle éventuellement substitué sur le noyau, soit un groupe hétéroaryl(C₁ ₋ ₃)alkyle éventuellement substitué sur le noyau.

La présente invention a également pour objet les intermédiaires de synthèse utilisés dans le procédé.

Le procédé selon l'invention est décrit dans le schéma 1:

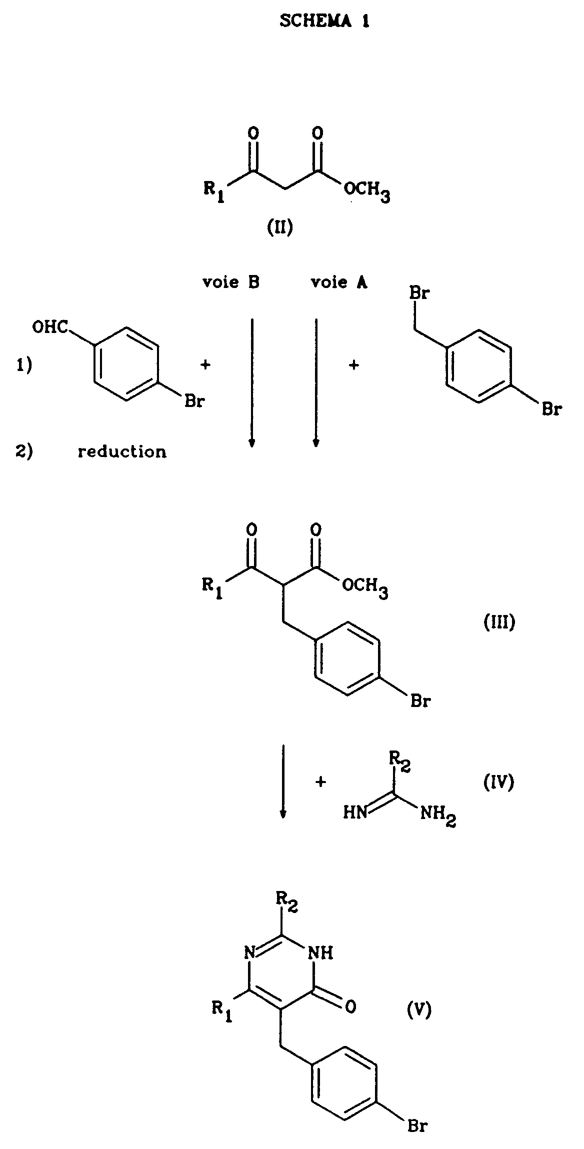

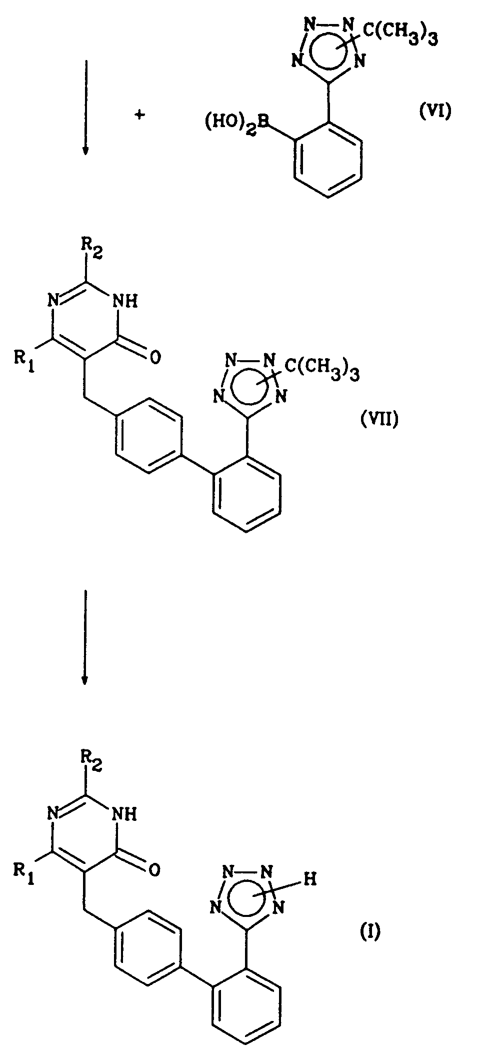

Dans une première étape,
■ soit on fait réagir un β-cétoester de formule générale (II) dans laquelle R₁ est tel que défini ci-dessus avec du 1-bromo-4-bromométhylbenzène (voie A) pour obtenir un composé de formule générale (III). La réaction est réalisée dans un solvant tel que le méthanol, l'éthanol, le 1,1-diméthyléthanol, le diméthylformamide, le diméthylsulfoxyde, l'acétonitrile ou la 1-méthylpyrrolidinone, en présence d'une base telle que l'hydrure de sodium, l'hydrure de lithium, le 1,1-diméthyléthylate de potassium ou un carbonate alcalin et éventuellement en présence d'un catalyseur tel que le bromure ou l'iodure de lithium, de magnésium ou de zinc.
■ soit on fait réagir un β-cétoester de formule générale (II) dans laquelle R₁ est tel que défini ci-dessus avec du 4-bromobenzaldéhyde (voie B) pour obtenir un dérivé bromé que l'on réduit en composé de formule générale (III). La première réaction est réalisée dans un solvant tel que le toluène, en présence d'une base telle que la pipéridine; la réduction est réalisée en présence de magnésium dans un solvant tel que le méthanol.

Dans une deuxième étape, on fait réagir un β-cétoester de formule générale (III) avec une amidine de formule générale (IV) dans laquelle R₂ est tel que défini ci-dessus pour obtenir une 5-[(4-bromophényl)méthyl)]-4-pyrimidinone de formule générale (V) dans laquelle R₁ et R₂ sont tels que définis précédemment. La réaction est conduite dans un solvant tel que le toluène.

Dans une troisième étape, on fait réagir une pyrimidinone de formule générale (V) avec un dérivé de l'acide boronique de formule (VI) pour obtenir un composé de formule générale (VII). La réaction est conduite dans un solvant tel que le toluène, en présence d'une base telle que le carbonate de sodium et d'un catalyseur tel que le palladium tétrakistriphénylphosphine.

Les composés de formule (VI) sont décrits dans la demande de brevet français déposée ce jour et ayant pour titre: "Dérivés de l'acide benzeneboronique, leur préparation et leur utilisation commme intermédiaires de synthèse".

Dans une quatrième étape, on réalise la déprotection du groupement tétrazole des composés de formule générale (VII), par chauffage dans un acide fort, comme par exemple l'acide bromhydrique aqueux, l'acide bromhydrique en solution dans de l'acide acétique, l'acide sulfurique ou l'acide acétique, en présence d'éthérate de trifluorure de bore, pour obtenir les composés de formule générale (I).

L'exemple suivant illustre l'invention.

Les analyses confirment la structure des produits obtenus.

### Exemple 1

6-butyl-2-(2-phényléthyl)-5-[[2'-(2*H*-tétrazol-5-yl)[1,1'-biphényl)-4-yl]méthyl]-pyrimidin-4(3*H*)-one.

### 1.1. 2-[(4-bromophényl)méthyl]-3-oxoheptanoate de méthyle

### Voie A:

Dans un ballon tricol de 250 ml, maintenu sous azote, on introduit 5,16 g de 1,1-diméthyléthylate de potassium et 60 ml de diméthylformamide. On refroidit le mélange à 4-5°C. On ajoute goutte à goutte, en 45 minutes, une solution de 7,28 g (46 mmoles) d'oxoheptanoate de méthyle dans 18 ml de diméthylformamide, puis 8,7 g de bromure de lithium. Après 15 minutes d'agitation, on additionne, en 45 minutes, une solution de 10 g (40 mmoles) de 1-bromo-4-bromométhylbenzène dans un mélange de diméthylformamide/tétrahydrofurane (1/1). On laisse une nuit à la température ambiante, on filtre et on évapore la solution sous vide. On reprend ensuite le résidu dans du dichlorométhane, on le lave à l'eau, on le séche sur du sulfate de magnésium puis on évapore à sec. On obtient une huile orange que l'on purifie par filtration sur silice. On réunit les fractions contenant le produit pur et on les évapore à sec, sous vide.

On obtient 8,6 g d'huile jaune. Le spectre RMN est compatible avec la structure du produit.
Rendement = 65,6 %

### Voie B:

a) 2-[(4-bromophényl)méthylène]-3-oxoheptanoate de méthyle Dans un ballon de 25 ml muni d'un Dean-Stark, on chauffe à la température de reflux, un mélange de 1,58 g (10 mmoles) de 3-oxoheptanoate de méthyle, 1,85 g (10 mmoles) de 4-bromobenzaldéhyde, 0,04 ml de pipéridine, 0,12 ml d'acide acétique et 10 ml de toluène. Après deux heures de chauffage, on récupère la quantité théorique d'eau et on évapore le toluène sous vide.
   On obtient 3,1 g de produit sous forme d'une huile jaune claire, mélange des deux isomères (Z et E).
   Rendement = 95,3 %
b) 2-[(4-bromophényl)méthyl]-3-oxoheptanoate de méthyle dans un ballon de 25 ml, on introduit 1,8 g (5,5 mmoles) du dérivé benzylidène obtenu précédemment en a), 10 ml de méthanol anhydre et 0,5 g de magnésium en tournures. On porte à la température de reflux et on laisse revenir le mélange réactionnel à la température ambiante. On le refroidit à 0°C et on le neutralise par addition lente d'acide acétique jusqu'à pH = 3. On filtre et on évapore sous vide. On reprend le résidu par de l'acétate d'éthyle, on le lave à l'eau, on le sèche et on l'évapore à sec.
   On obtient 1,6 g d'huile jaune. Le spectre RMN est compatible avec la structure du produit.
   Rendement = 88,4 %

### 1.2. 5-[(4-bromophényl)méthyl]-6-butyl-2-(2-phényléthyl)pyrimidin-4(3H)-one.

Dans un ballon de 50 ml, on introduit 8,4 g (25,67 mmoles) du cétoester obtenu précédemment et 17 ml de toluène. On porte la solution au reflux. On ajoute, par petites portions, en 4 heures, 6,5 g (43,86 mmoles) de benzène-3-propanamidine. Au bout de 4 heures 30 on évapore le mélange à sec, sous vide, et on triture le résidu dans 30 ml de 1,1-diméthyléthylméthyléther. On filtre le solide, on le lave au 1,1-diméthyléthylméthyléther glacé et on le sèche sous vide. On obtient 7 g de produit.
Point de fusion = 159-161,5°C Rendement = 64,2 %

### 1.3. 6-butyl-5-[[2'-[2-(1,1-diméthyléthyl)-2H-tétrazol-5-yl]-[1,1'-biphényl]-4-yl]méthyl]-2-(2-phényléthyl)pyrimidin-4(3H)-one

Dans un ballon bicol, muni d'un réfrigérant, on introduit successivement, 1,2 g (4,8 mmoles) d'acide 2-[2-(1,1-diméthyléthyl)-2*H*-tétrazol-5-yl]benzèneboronique, 2,07 g (4,8 mmoles) du dérivé bromé obtenu précédemment, 0,28 g (0,24 moles) de palladium tétrakistriphénylphosphine, 5 ml d'une solution de carbonate de sodium 2 M et 25 ml de toluène. On porte ce mélange à la température de reflux pendant 16 heures. Après refroidissement et décantation, on extrait la phase aqueuse avec 150 ml d'acétate d'éthyle. On rassemble les phases organiques et on les lave successivement avec 20 ml d'eau puis avec 20 ml d'une solution saturée de chlorure de sodium. On les sèche sur du sulfate de magnésium. Après évaporation du solvant, on purifie le résidu par chromatographie sur colonne de gel de silice en éluant par un mélange acétate d'éthyle/hexane (1/4).

On obtient 1,42 g de produit sous forme d'un solide blanc.
Point de fusion = 143-145°C Rendement = 53,4 %

### 1.4. 6-butyl-2-(2-phényléthyl)-5-[[2'-(2H-tétrazol-5-yl)[1,1'-biphényl]-4-yl]méthyl]-pyrimidin-4(3H)-one.

Dans un ballon de 25 ml on introduit 1 g du dérivé obtenu précédemment en 1.3 dans 10 ml d'acide bromhydrique à 60%. On chauffe le mélange 20 minutes à 120 °C puis on le verse sur de l'eau glacée. On lave le précipité obtenu à l'eau et on le dissout dans un volume minimum de méthanol. On basifie le mélange par du bicarbonate de sodium aqueux, on le dilue avec de l'eau et on neutralise par de l'acide acétique. On filtre, on lave à l'eau et au méthanol. On sèche sous vide.

On obtient 0,8 g du composé attendu.
Point de fusion = 227 °C Rendement = 89,3 %

Le procédé selon l'invention permet d'obtenir les composés (I) avec un bon rendement.

Les intermédiaires de formule générale (V) 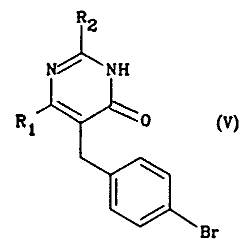
et de formule générale (VII) 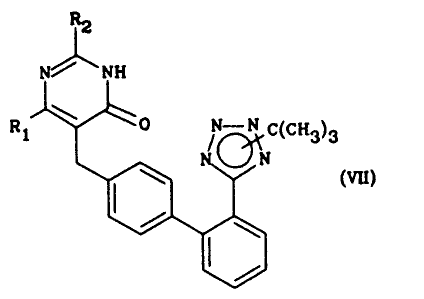
dans lesquelles R₁ et R₂ sont tels que définis à la page 1, font également partie de l'invention.

## Revendications

1. Procédé de préparation des composés répondant à la formule générale (I) 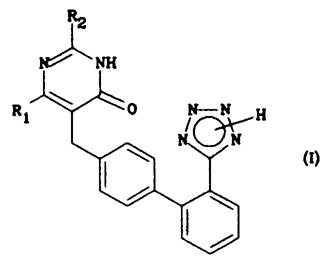 dans laquelle
R₁ représente soit un groupe (C₁ ₋ ₇)alkyle droit ou ramifié, soit un groupe (C₃ ₋ ₉)alcényle droit ou ramifié, soit un groupe cyclo(C₃ ₋ ₇)alkyl(C₁ ₋₆)alkyle,
R₂ représente soit un atome d'hydrogène, soit un groupe (C₁ ₋ ₇)alkyle droit ou ramifié, soit un groupe cyclo(C₃ ₋ ₇)alkyl(C₁ ₋ ₃)alkyle, soit un groupe aryl(C₁ ₋ ₃)alkyle éventuellement substitué sur le noyau, soit un groupe aryloxy(C₁ ₋ ₃)alkyle éventuellement substitué sur le noyau, soit un groupe arylthio(C₁ ₋ ₃)alkyle éventuellement substitué sur le noyau, soit un groupe arylsulfonyl(C₁₋₃)alkyle éventuellement substitué sur le noyau, soit un groupe hétéroaryl(C₁ ₋ ₃)alkyle éventuellement substitué sur le noyau, procédé caractérisé en ce que l'on fait réagir un composé de formule générale (II) 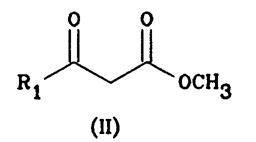
■ soit avec du 1-bromo-4-bromométhylbenzène,
■ soit avec du 4-bromobenzaldéhyde et on réduit le composé formé
pour obtenir un composé de formule générale (III) 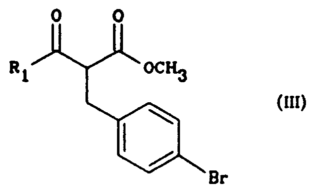 que l'on fait réagir avec une amidine de formule générale (IV) 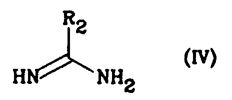 pour obtenir un composé de formule générale (V) 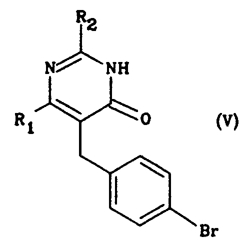 que l'on fait réagir avec un composé de formule générale (VI) 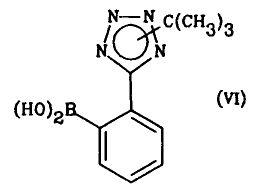 pour obtenir un composé de formule générale (VII) 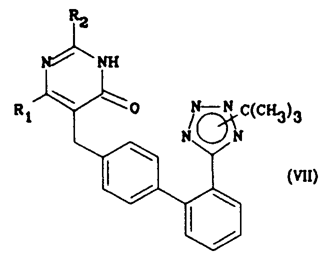 que l'on déprotège en composé de formule générale (I)

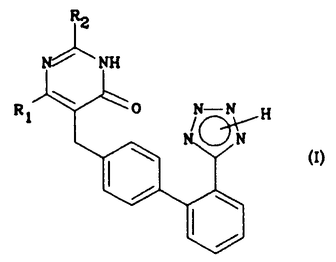

2. Procédé selon la revendication 1 caractérisé en ce que la réaction entre le composé (II) et le 1-bromo-4-bromométhylbenzène est effectuée dans un solvant tel que le diméthylformamide, en présence d'une base telle que le 1,1-diméthylate de potassium et éventuellement d'un catalyseur tel que le bromure de lithium.

3. Procédé selon la revendication 1 caractérisé en ce que la réaction entre le composé (II) et le 4-bromobenzaldéhyde est conduite dans un solvant tel que le toluéne, en présence d'une base telle que la pipéridine.

4. Procédé selon la revendication 1 caractérisé en ce que la réduction qui conduit au composé (III) est réalisée par du magnésium dans un solvant tel que le méthanol.

5. Procédé selon la revendication 1 caractérisé en ce que la réaction entre le composé (III) et le composé (IV) est effectuée dans un solvant tel que le toluène.

6. Procédé selon la revendication 1 caractérisé en ce que la réaction entre le composé (V) et le composé (VI) est réalisée dans un solvant tel que le toluène, en présence d'une base telle que le carbonate de sodium et d'un catalyseur tel que la palladium tétrakistriphénylphosphine.

7. Composés de formule générale (V) 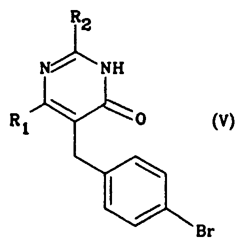 dans laquelle R₁ et R₂ sont tels que définis dans la revendication 1, en tant qu'intermédiaires de synthèse du procédé selon la revendication 1.

8. Composés de formule générale (VII) 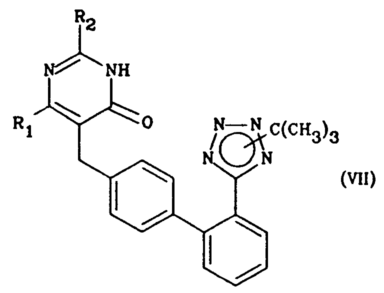 dans laquelle R₁ et R₂ sont tels que définis dans la revendication 1, en tant qu'intermédiaires de synthèse du procédé selon la revendication 1.
